# EUROPÄISCHE PATENTANMELDUNG

(11) **EP 3 928 866 A1**
(43) Veröffentlichungstag der Anmeldung: **29.12.2021**
(21) Anmeldenummer: 21191874.3
(22) Anmeldetag: 01.04.2014
(51) Int. Cl.: B01L 3/00, B65D 51/16, A61M 39/10, A61J 1/20, B65D 41/06

(54) **VERSCHLUSS FÜR EINEN BEHÄLTER**

(30) Priorität: 03.04.2013 EP 13162169
(62) Teilanmeldung aus: 14713863.0
(71) Anmelder: Metrohm AG, 9100 Herisau (CH); Sigma-Aldrich Chemie GmbH, 9471 Buchs (CH)
(72) Erfinder: Christensen, Björn, 9000 St. Gallen (CH); Wahl, Fabian, 9000 St. Gallen (CH); Weber, Michael, 9000 St. Gallen (CH); Schindler, Samuel, 8048 Zürich (CH); Wilhelm, Lukas, 8048 Zürich (CH)
(74) Vertreter: Hepp Wenger Ryffel AG

(57) **Zusammenfassung**

Verschluss (1) für einen Behälter, der ein Verbindungselement (2) zur Verbindung des Verschlusses mit einem Behälter, eine Versiegelungsvorrichtung (3) zum Versiegeln des Zugangs zu einem Behälterinhalt und eine Schnittstelle (4) zu einem Adapter mit einer Kupplungsaufnahme (5) umfasst. Der Verschluss (1) weist eine Lieferposition und eine Gebrauchsposition auf, wobei in der Lieferposition die Versiegelungsvorrichtung (3) gas- und flüssigkeitsdicht verschlossen ist. In einer Gebrauchsposition ist die Versiegelungsvorrichtung (3) geöffnet.

## Beschreibung

Die Erfindung betrifft einen Verschluss für einen Behälter, einen Adapter für einen Verschluss, ein Adaptersystem, ein Verfahren zum Entnehmen eines Fluids aus einem Behälter, ein Anzeigeelement zur Anzeige eines Funktionszustandes, eine Versiegelungsvorrichtung sowie die Verwendung eines Adapters und/oder eines Verschlusses gemäss den Oberbegriffen der unabhängigen Ansprüche.

Insbesondere im Rahmen von Laboranwendungen ist die Prozesssicherheit sowie die Prozessqualität bei der Verwendung von verschiedensten Fluiden essenziell wichtig. Des Weiteren ist es vorteilhaft, wenn der Bedienkomfort für den Anwender erhöht ist, welches ebenfalls zur Prozesssicherheit beiträgt.

Bisherige Verschlüsse von Behältern für Fluide im Laborbereich ermöglichen ein sicheres Verschliessen und Öffnen des Behälters, jedoch können Verunreinigungen durch beispielsweise Luftzufuhr entstehen. Des Weiteren ist die Verbindung eines Behälters mit einem Gerät, welches das Fluid verwendet, oftmals kompliziert herzustellen, wodurch die Handhabung erschwert ist.

Es ist daher Aufgabe der vorliegenden Erfindung, die Nachteile des Standes der Technik zu vermeiden und insbesondere einen Verschluss, einen Adapter, ein Verfahren zum Entnehmen eines Fluids aus einem Behälter, ein Anzeigeelement, eine Versiegelungsvorrichtung sowie die Verwendung eines Adapters und/oder eines Verschlusses zu schaffen, die eine leichte und sichere Entnahme eines Fluids aus einem Behälter ermöglichen und den Aufwand und die Fehlerquellen eines Anwenders verringern.

Zur Lösung der Aufgabe führt ein Verschluss für einen Behälter, der ein Verbindungselement zur Verbindung des Verschlusses mit einem Behälter und eine Versiegelungsvorrichtung zum Versiegeln des Zugangs zu einem Behälterinhalt umfasst. Des Weiteren umfasst der Verschluss eine Schnittstelle zu einem Adapter mit einer Kupplungsaufnahme, wobei der Verschluss eine Lieferposition und eine Gebrauchsposition aufweist. In der Lieferposition ist die Versiegelungsvorrichtung gas- und flüssigkeitsdicht verschlossen, wobei die Versiegelungsvorrichtung in einer Gebrauchsposition geöffnet ist. Kanäle zur Zuführung und Abführung von Luft durch die Versiegelungsvorrichtung sind verschliessbar ausgebildet. Ein mit einem solchen Verschluss ausgestatter Behälter ist nach aussen hin im Wesentlichen gas- und flüssigkeitsdicht verschlossen, wie nachstehend noch im Detail erläutert ist.

Die Kanäle sind hierbei bevorzugt gegen Aussen und gegen Innen verschlossen, wobei die Versiegelungsvorrichtung und die Kanäle bevorzugt Bestandteil des Verschlusses sind.

Die Kanäle können in einem geschlossenen und in einem offenen Zustand Luft durch ein Filterelement leitbar machen. Im geschlossenen Zustand, also in der Lieferposition, ist hierbei ein geschlossener Luftkreislauf durch die Kanäle und das Filterelement ausgebildet, der keinen Kontakt zu Aussenluft ermöglicht.

Weiterhin ermöglicht ein derartiger Verschluss das kontaminations- und leckagefreie Transportieren eines Fluids in einem Behälter sowie das leichte Verbinden mit einem Adapter, wobei der Verschluss beim oder vor dem Verbinden mit einem Adapter geöffnet wird.

Die Versiegelungsvorrichtung verschliesst die Kanäle insbesondere bevorzugt in der Lieferposition mit geschlossener Versiegelung.

Definierte Kanäle zur Zu- und Abführung von Luft ermöglichen ein besonders sicheres und kontaminationsfreies Arbeiten. Die Luft wird bevorzugt durch ein Filterelement geleitet, bevor sie mit dem Behälterinhalt in Kontakt kommt. Des Weiteren sind die Kanäle in einer Lieferposition bevorzugt verschlossen und werden erst in einer Gebrauchsposition geöffnet.

Die Versiegelungsvorrichtung umfasst bevorzugt eine Versiegelungsvorrichtung wie nachfolgend beschrieben.

Die Lieferposition des Verschlusses umfasst eine verschlossene Versiegelung sowie bevorzugt einen Verschluss von Luftzuführungs- und Luftabführungskanälen.

In der Gebrauchsposition ist die Versiegelung geöffnet und ein Fluid aus einem Behälter kann durch den Verschluss entnommen werden. Bevorzugt kommt das Fluid jedoch nicht mit Aussenluft direkt in Verbindung, wie nachfolgend noch erläutert wird.

Das Verbindungselement kann ein Gewinde umfassen.

Ein Gewinde ermöglicht das Befestigen des Verschluss einfach und unkompliziert auf ein passendes Gegengewinde eines Behälters. Des Weiteren sind selbstverständlich auch Bajonettverschlüsse oder Schnappverschlüsse möglich.

Der Verschluss kann ein Deckelelement zum Öffnen und Verschliessen des Verschlusses umfassen, wobei das Deckelelement bevorzugt durch ein Bruchelement sicherbar ist, welches ein erstmaliges Öffnen anzeigt.

Ein derartiges Deckelelement ermöglicht ein Verschliessen und Wiederöffnen des Verschlusses nach dem Öffnen der Versiegelungsvorrichtung in einer Gebrauchsposition. Ein weiteres Bruchelement an dem Deckelelement zum Anzeigen eines erstmaligen Öffnens macht den Verschluss manipulierungssicher.

Der Verschluss kann ein Filterelement umfassen, durch welches, insbesondere in der Gebrauchsposition, Aussenluft von aussen durch den Verschluss in einen Behälter leitbar ist.

Um Fluid aus dem Behälter entnehmen zu können, ist eine Luftzufuhr von Vorteil, um einen Druckausgleich zu ermöglichen. Der Einsatz eines Filterelements verhindert die Kontamination des Inhalts des Behälters durch direkte Aussenluft und ermöglicht somit ein genaues und qualitativ hochwertiges Arbeiten.

Filterelemente sind bevorzugt mit einem Material ausgestattet, welches dazu geeignet ist, Anteile aus der Umgebungsluft zu entfernen, welche eine Veränderung des Behälterinhalts bewirken können. Das jeweilige Material wird hierbei in fachüblichen Routinemassnahmen auf den jeweiligen Behälterinhalt abgestimmt. Typische Beispiele geeigneter Materialien sind Atemkalk (eine auch als Natronkalk bekannte Mischung aus Calciumhydroxid Ca(OH)₂ und Natriumhydroxid NaOH; oder auch eine Mischung aus Kaliumhydroxid KOH und Bariumhydroxid Ba(OH)₂); Partikelfilter; Molekularsiebe, Silicagel.

Das Filterelement kann lösbar ausgestaltet sein. Bevorzugt ist das Filterelement in einer Gebrauchsposition lösbar ausgestaltet. Somit kann das Filterelement ausgetauscht werden und der jeweiligen Situation angepasst werden.

Die Kanäle zur Zuführung- und Abführung von Luft in dem Verschluss können, zumindest an einer Anschlussseite zum Filterelement, besonders bevorzugt als weibliche Lueranschlüsse (mit einem Innenkonus) oder mit einem Gewinde ausgestattet sein. So kann im Falle eines abnehmbaren Filterelements auch ohne angeschlossenes Filterelement bspw. ein Schlauch für die Zufuhr von Schutzgas besonders einfach an dem Verschluss angebracht werden.

Das Filterelement kann weiterhin abnehmbar und bevorzugt austauschbar ausgebildet sein.

Somit ist eine Wahl des Filtermediums möglich und der Verschluss ist sehr vielseitig verwendbar.

Weiterhin kann das Filterelement einen Filterweg umfassen, der eine chemische Reaktion des Filtermaterials mit der das Filtermaterial durchströmenden Luft ermöglicht. Ein Filterweg ist hierbei im Rahmen der Anmeldung definiert als Abstand von Lufteinlass in den Filterelement sowie Luftauslass aus dem Filterelement.

Der Verschluss kann ein Fixierungselement umfassen, welches den Verschluss in einer Lieferposition fixiert, wobei das Fixierungselement lösbar ausgebildet ist. Das Fixierungselement ist insbesondere bevorzugt zerstörbar lösbar ausgebildet und nach der Lösung und bevorzugt Entfernung des Fixierungselements ist der Verschluss in eine Gebrauchsposition bringbar.

Ein derartiges Fixierungselement verhindert ein vorzeitiges Erreichen der Gebrauchsposition des Verschlusses, falls dies noch nicht gewollt ist. Des Weiteren stellt ein derartiges Fixierungselement eine visuelle Anzeige des Öffnungszustandes und/oder der Liefer- beziehungsweise Gebrauchsposition des Verschlusses dar. Somit wird ein fehlerfreies Arbeiten leicht ermöglicht.

Der Verschluss kann einen Datenträger umfassen, bevorzugt einen Datenträger mit zerstörbarem Datenübertragungselement, insbesondere bevorzugt einen RFID-Chip.

Ein Datenträger ermöglicht die automatische Erkennung des Verschlusses sowie der Daten, die auf dem Datenträger gespeichert sind, wie beispielsweise Daten des zugehörigen Behälters sowie des Inhalts des Behälters. Dies führt zu einer sicheren Arbeitsweise, da wichtige Daten übertragen werden können. So kann beispielsweise der Inhalt des Behälters fehlerfrei erkannt werden. Des Weiteren können Daten wie beispielsweise Volumen, Haltbarkeitsdaten oder entnommene Mengen über den Datenträger festgestellt beziehungsweise auf diesem gespeichert werden. Dies führt zu qualitativ hochwertigen Resultaten. Der Datenträger kann optisch und/oder elektronisch ausgebildet sein.

Die Kupplungsaufnahme kann entlang einer Längsachse des Verschlusses von einer Lieferposition in eine Gebrauchsposition bewegbar ausgebildet sein.

Durch eine Bewegbarkeit der Kupplungsaufnahme kann die Gebrauchsposition bei dem Einkuppeln eines passenden Kupplungselements für die Kupplungsaufnahme erreicht werden. Das Öffnen der Versiegelungsvorrichtung kann insbesondere unabhängig vom Einsatz des Adapters erfolgen, vorzugsweise durch einfaches Herunterdrücken des Verschlusses, wobei ein passender Adapter dann in einem nachfolgenden Schritt angebracht wird.

Der Verschluss ist in einer Lieferposition bevorzugt gasdicht und flüssigkeitsdicht verschlossen. In einer Gebrauchsposition kann Fluid aus dem Behälter durch den Verschluss geführt werden. Nichtsdestotrotz bleibt die Gaszufuhr, beziehungsweise Luftzufuhr durch ein Filterelement kontrolliert, so dass keine Kontaminationen des Inhalts des Behälters auftreten können.

Die Versiegelungsvorrichtung kann nach Entfernen des Fixierungselements und Herunterdrücken des Deckels geöffnet, bevorzugt gebrochen werden, wobei der Verschluss nach dem Aufbrechen der Versiegelungsvorrichtung in der Gebrauchsposition fixierbar ist.

Somit bleibt die Kupplungsaufnahme nach dem Aufbrechen der Versiegelung in einer Gebrauchsposition und ist erkennbar für den Benutzer bereits gebraucht. Dies erhöht die Sicherheit und macht die Verwendung des Verschlusses für den Anwender einfach und zuverlässig.

In der Gebrauchsposition ist die insbesondere durch ein Filterelement gereinigte Luft durch den Verschluss leitbar.

Die Zuführung von Luft ermöglicht einen Druckausgleich und erleichtert somit das Entnehmen von Fluid aus dem Behälter unter kontaminationsfreien Bedingungen.

Die Kupplungsaufnahme kann Eingriffsnuten umfassen, bevorzugt acht Eingriffsnuten. Die Ausbildung von Eingriffsnuten ermöglicht ein passgenaues Einführen eines Kupplungselements sowie eine dezidierte Ausrichtung eines Adapters auf dem Verschluss. Des Weiteren ist die Kraftübertragung von Adapter auf den Verschluss optimiert. Die Eingriffsnuten weisen bevorzugt ausschliesslich eine Führungsfunktion für ein Kupplungselement eines Adapters auf. Insbesondere weisen die Eingriffsnuten keine Haltefunktion zum Fixieren eines Adapters in einem gekoppelten Zustand von Verschluss und Adapter auf. Die Eingriffsnuten führen ausschliesslich zu einer Verhinderung einer Drehbewegung, nicht jedoch für eine Bewegung in oder entgegen der Einführungsrichtung.

Die Versiegelungsvorrichtung kann mindestens eine Sollbruchstelle, bevorzugt genau zwei Sollbruchstellen aufweisen, die bevorzugt ein simultanes Aufbrechen der Versiegelung des Fluids in einem Behälter sowie der Versiegelung der Kanäle zur Luftzufuhr und -abfuhr ermöglicht.

Somit wird ein besonders sauberes, prozesssicheres Arbeiten ermöglicht, da auch die Luftkanäle zunächst versiegelt bleiben, bis sie benötigt werden.

Der Verschluss kann weiterhin mindestens zwei Dichtungen umfassen. Bevorzugt umfasst der Verschluss eine erste Dichtung zum Abschluss der Luftkanäle gegen Aussenluft in der Gebrauchsposition und eine zweite Dichtung zum Abschluss des zu entnehmenden Fluids an einer Entnahmevorrichtung, wie beispielsweise einem Entnahmeschlauch, in der Gebrauchsposition.

Der Verschluss kann zumindest ein Fixierungselement umfassen, an welchem ein Adapter befestigbar, bevorzugt lösbar befestigbar, ist.

Ein derartiges Fixierungselement kann eine Hinterschneidung oder Kante oder Haltefläche umfassen, die von Eingriffselementen eines Adapters umgriffen werden können. Somit ist ein Adapter sicher auf dem Verschluss fixiert und positioniert.

Der Verschluss kann zur mehrfachen Verwendung ausgebildet sein und hierfür insbesondere keine von der Gebrauchsposition verschiedene Lieferposition aufweisen. Ein derartiger Verschluss kann so ausgebildet sein, dass er kein Fixierungselement zur Fixierung des Verschlusses in einer Lieferposition aufweist.

Ein derartiger Verschluss ist mit einem Adapter leicht verbindbar und kann mit verschiedenen Behältern und/oder Adaptern verwendet werden. Dies optimiert die Kosten in einem Labor.

Zur Lösung der Aufgabe führt weiterhin ein Behälter, der mit einem Verschluss wie vorhergehend beschrieben verbunden, bevorzugt nach aussen hin im Wesentlichen gas- und flüssigkeitsdicht verschlossen ist.

Ein derartiger Behälter kann leicht und sicher mit einem Adapter verbunden werden und ermöglicht ein sicheres Aufbewahren sowie kontaminationsfreies und leichtes Entnehmen eines Fluids aus dem Behälter.

Zur Lösung der Aufgabe führt weiterhin ein Adapter für einen Verschluss, bevorzugt einen Verschluss wie vorhergehend beschrieben, der eine Verbindungsstelle zu einem Gerät, bevorzugt eine Auswertungseinheit und eine Schnittstelle zu einem Verschluss umfasst. Die Schnittstelle umfasst weiterhin ein Kupplungselement, welches mit einer Kupplungsaufnahme eines Verschlusses in Eingriff bringbar ist, so dass ein Fluidstrom durch den Adapter erzeugbar ist, ohne einen direkten Kontakt von Aussenluft und Fluid. Das Kupplungselement ist relativ zum Adapter entlang der Achse in einer Einführungsrichtung des Adapters in einen Verschluss bewegbar ausgebildet.

Ein derartiger Adapter ermöglicht eine sichere und einfache Verbindung eines Verschlusses mit einem Adapter, um ein Fluid aus einem Behälter sicher und einfach zu entnehmen.

Der Adapter weist bevorzugt ausschliesslich einen wirksamen Kanal für ein Medium aus einem Behälter auf, bevorzugt im Inneren des Kupplungselements. Somit bleibt das Medium auf seinem Weg durch den Adapter kontminationsfrei.

Ein wirksamer Kanal bedeutet im Rahmen der Erfindung, dass jeweils eine Fluidmenge durch den Kanal in eine Richtung fliessen kann, auch wenn sich weitere Unterteilungselement im Kanal befinden.

Ein derartiger Adapter ermöglicht das kontaminationsfreie und leichte Entnehmen eines Fluid aus einem Behälter und das Weiterführen in ein Gerät, welches das Fluid benötigt. Der Arbeitsaufwand für einen Benutzer wird vereinfacht und verringert und die Prozesssicherheit steigt.

Das Kupplungselement kann Eingriffselemente umfassen, bevorzugt acht Eingriffselemente, die in Eingriffnuten einer Kupplungsaufnahme eines Verschlusses einführbar sind, wie nachfolgend noch im Detail anhand von Ausführungsbeispielen erläutert wird. Die Eingriffsnuten sind bevorzugt an einem sich konisch in Richtung eines Behälters verjüngenden Bereich der Kupplungsaufnahme angeordnet.

Die Ausbildung von Eingriffelementen ermöglicht ein pass- und orientierungsgenaues, einfaches und sicheres Einführen des Kupplungselements in die Kupplungsaufnahme eines Verschlusses und somit ein sichere Verbindung von Adapter und Verschluss.

Besonders bevorzugt sind die Eingriffsnuten einer Kupplungsaufnahme eines Verschlusses in einem sich konisch in Richtung des Behälters verjüngenden Bereich dieser Kupplungsaufnahme angeordnet. Besonders bevorzugt laufen diese Eingriffsnuten im weiteren Teil des sich konisch in Richtung des Behälters verjüngenden Bereichs flach aus. Es hat sich gezeigt, dass hierdurch ein besonders vorteilhafter Kompromiss aus einfacher Handhabbarkeit und Funktionssicherheit sichergestellt werden kann.

Das Kupplungselement kann relativ zum Adapter bewegbar ausgebildet sein, bevorzugt entlang der Achse einer Einführungsrichtung des Adapters in einen Verschluss bewegbar.

Ein bewegbares Kupplungselement ermöglicht die passgenaue Verbindung zwischen Kupplungselement und Kupplungsaufnahme unabhängig von der Befestigung des Adapters am Verschluss. Des Weiteren wird eine zuverlässige Dichtung zwischen dem Kupplungselement und einem Fluidentnahmeschlauch des Verschlusses durch Flächenpressung ermöglicht. Der Fluidentnahmeschlauch ist hierbei auskragend ausgebildet, sodass das Kupplungselement auf dem Kragen zum Anliegen kommt. Auf ein zusätzliches Dichtungselement kann daher in besonders vorteilhafter Weise verzichtet werden.

Der Adapter kann Hebelelemente umfassen, durch die das Kupplungselement relativ zum Adapter bewegbar ist. Die Verwendung von Hebelelementen definiert die Beweglichkeit des Kupplungselements relativ zum Adapter und ermöglicht somit ein passgenaues Verbinden von Adapter und Verschluss.

Der Adapter kann Befestigungselemente umfassen, die mit einem Fixierungselement eines Verschlusses in Eingriff bringbar sind, wobei die Befestigungselemente bevorzugt mechanisch lösbar ausbildet sind. Insbesondere bevorzugt sind die Befestigungselemente während der Bewegung des Kupplungselements lösbar ausgebildet.

Die Befestigungselemente ermöglichen eine passgenaue Fixierung des Adapters auf einem Verschluss und ein Lösen. Die Befestigungselemente sind bevorzugt Schnappelemente, die auf eine Hinderschneidung eines Verschlusses eingreifen. Somit ist der Adapter sicher mit dem Verschluss verbunden.

Die Verbindungsstelle kann eine Gewindekupplung umfassen.

Ein Gewindekupplung ermöglicht eine schnelle und sichere Verbindung des Adapters mit einem Gerät, welches Fluid aus einem Behälter mit einem Verschluss benötigt.

Der Adapter kann ein Ausleselement für Daten umfassen, bevorzugt eine Ausleseelektronik für einen RFID-Chip.

Durch ein Ausleseelement am Adapter kann der Adapter Daten des Verschlusses und somit, falls eingespeichert, auch des Behälters und des im Behälter befindlichen Fluids auslesen. Dies führt zu höherer Prozesssicherheit, da Fehler durch den Benutzer ausgeschlossen sind.

Das Ausleseelement kann eine Schreibfunktion für Daten aufweisen. Eine derartige Schreibfunktion ermöglicht das Kennzeichnen des Verschlusses und somit auch des Behälters durch den Adapter, so dass beispielsweise Datum der Benutzung, Zeitpunkte von An- und Abkoppeln des Verschlusses oder Öffnen oder Verbrauch an Fluid festgehalten werden können.

Dies führt zu erhöhter Prozesssicherheit.

Der Adapter kann ein Anzeigelement aufweisen. Insbesondere kann der Adapter ein Anzeigelement aufweisen wie nachfolgend beschrieben. Ein derartiges Anzeigelement ermöglicht das Anzeigen einer Verbindung von Adapter und Verschluss.

Zur Lösung der Aufgabe führt weiterhin ein Adaptersystem für einen Behälter, der einen Verschluss sowie einen Adapter wie vorhergehend beschrieben umfasst.

Zur Lösung der Aufgabe führt weiterhin ein Verfahren zum Entnehmen eines Fluid aus einem Behälter, wobei das Fluid durch ein Adaptersystem wie vorhergehend beschrieben geführt wird.

Ein derartiges Verfahren ermöglicht die sichere und zuverlässige Entnahme von Fluid aus einem Behälter unter kontaminationsfreien Bedingungen.

Zur Lösung der Aufgabe führt weiterhin ein Anzeigelement zur Anzeige eines Funktionszustandes, insbesondere eines Kopplungszustandes zwischen einem Verschluss wie vorgehend beschrieben und einem Adapter wie vorhergehend beschrieben. Das Anzeigeelement umfasst zumindest ein Anzeigekörper sowie zumindest eine Lichteinkopplungsvorrichtung zum Einkoppeln von Licht in den Anzeigekörper. Des Weitern sind Lichtlenkelemente zum Verteilen des eingekoppelten Lichts in dem Anzeigekörper ausgebildet, die das eingekoppelte Licht im Anzeigekörper verteilen.

Ein derartiges Anzeigelement zeigt deutlich und zuverlässig den Kopplungszustand zwischen Verschluss und Adapter an, und führt somit zur erhöhten Verwendungssicherheit von Adapter und Verschluss.

Die Lichtlenkelemente können Grenzflächen umfassen.

Durch die Verwendung von Grenzflächen wird das Licht gebrochen und somit umgelenkt. Weiterhin sind Grenzflächen einfach und günstig herzustellen. Das Licht wird somit durch den gesamten Anzeigekörper geleitet und die Anzeige ist somit seht deutlich und leicht zu erkennen.

Grenzflächen im Rahmen der Anmeldung sind Flächen, an denen sich der Brechungsindex ändert. Alternativ können selbstverständlich auch andere oder zusätzliche reflektive und/oder diffraktive Elemente verwendet werden.

Der Anzeigekörper kann eine Oberseite und eine gegenüberliegende Unterseite umfassen, wobei die Oberseite kreisförmig ausgebildet ist und die Unterseite im Vergleich zur Oberseite in einem Winkel von etwa 8° bis etwa 20°, bevorzugt von etwa 10° bis etwa 15°, besonders bevorzugt von etwas 12° angeordnet ist.

Eine derartige Ausbildung des Anzeigekörpers verbessert die Verteilung des Lichts im Anzeigekörper, insbesondere in der vollständigen dreidimensionalen Geometrie des Anzeigekörpers, so dass die Anzeige aus allen Richtungen sichtbar ist.

Zur Lösung der Aufgabe führt weiterhin eine Versiegelungsvorrichtung zum Versiegeln eines Behälters, die Bruchelemente zum endgültigen Aufbrechen der Versiegelung durch ein Aufbrechelement zum Durchstossen des Bruchelements in eine Durchstossrichtung umfasst. Des Weiteren umfasst die Versiegelungsvorrichtung ein Fluidanschlusselement zum Herstellen einer Verbindung zu einem Fluid nach dem Aufbrechen der Versiegelung. Zumindest ein Teil des Bruchelements ist entlang der Durchstossrichtung von einer Lieferposition in eine Gebrauchsposition verschiebbar ausgebildet, wobei das Bruchelement bevorzugt in der Lieferposition eine Sollbruchstelle aufweist.

Die Sollbruchstelle ist bevorzugt am Umfang des Bruchelements angeordnet, so dass nach dem Aufbrechen der Sollbruchstelle das Bruchelement weniger Höhenausdehnung aufweist als in einem Lieferzustand. Die Kraft zum Brechen der Sollbruchstelle ist bevorzugt manuell aufbringbar, etwa in einem Bereich von 15N bis 30N, vorzugsweise etwa 17N bis 25N, besonders bevorzugt etwa 20N. In besonders bevorzugten Ausführungsformen führt ein darüber hinausgehender Druck zu einem fühlbaren Einschnappen; der hierfür aufzuwendende Druck sollte etwa in einem Bereich von 30N bis 50N, vorzugsweise etwa 35N bis 45N, besonders bevorzugt bei etwa 40N liegen. Durch diese haptische Rückkopplung wird die Handhabbarkeit weiter verbessert.

Eine derartige Ausbildung der Versiegelungsvorrichtung ermöglicht ein sicheres Anzeigen des Öffnungszustandes und gleichzeitig eine optimale Positionierung des Fluidanschlusselements zur Erstellung einer Fluidverbindung zwischen Fluidanschlusselement und einem Kupplungselement.

Das Bruchelement kann nach dem Aufbrechen der Sollbruchstelle einen ersten verschiebbaren und einen zweiten fixen Teil umfassen. Der erste verschiebbare Teil ist in Durchstossrichtung verschiebbar.

Somit ist ein vollständiges Brechen der Sollbruchstelle entlang des gesamten Umfangs klar erkennbar. Dies führt zu einer verbesserten Prozesssicherheit, da die Höhe der Versiegelungsvorrichtung sich entlang des gesamten Umfangs ändert und somit detektierbar ist.

Das Bruchelement kann weiterhin Siegelklappen aufweisen, die zwischen einander Sollbruchstellen aufweisen und bevorzugt jeweils dreieckig ausgebildet sind.

Das Vorhandensein von Siegelklappen ermöglicht den endgültigen Zugang zu dem Inneren der Versiegelungsvorrichtung und ermöglichen so in einer Lieferposition eine gas- und flüssigkeitsdichte Ausbildung der Versiegelungsvorrichtung sowie in einer Gebrauchsposition das leichte Einführen eines Kupplungselements zur Entnahme eines Fluid aus einem Behälter.

Dreieckige Ausbildung der Siegelklappen bedeutet im Rahmen der Erfindung, dass die Siegelklappen zumindest drei Ecken aufweisen. Es ist hierbei möglich dass die Sehnen des Dreiecks gerundet, gerade oder elliptisch ausgebildet sind.

Ein Adapter wie vorhergehend beschrieben und/oder eine Verschluss wie vorhergehend beschrieben können zum Hindurchführen eines Fluid bei der Entnahme aus einem Behälter verwendet werden.

Zur Lösung der Aufgabe führt weiterhin ein Verschluss für einen Behälter, der ein Verbindungselement zur Verbindung des Verschlusses mit einem Behälter und eine Schnittstelle zu einem Adapter mit einer Kupplungsaufnahme umfasst. Der Verschluss umfasst weiterhin ein Filterelement, durch welches Aussenluft von aussen durch den Verschluss in einen Behälter leitbar ist, wobei das Filterelement ein Filtervolumen bildet, welches mit Filtermaterial auffüllbar ist.

Um Fluid aus dem Behälter entnehmen zu können, ist eine Luftzufuhr von Vorteil, um einen Druckausgleich zu ermöglichen. Der Einsatz eines Filterelements verhindert die Kontamination des Inhalts des Behälters durch direkte Aussenluft und ermöglicht somit ein genaues und qualitativ hochwertiges Arbeiten.

Filterelemente sind bevorzugt mit einem Material ausgestattet, welches dazu geeignet ist, Anteile aus der Umgebungsluft zu entfernen, welche eine Veränderung des Behälterinhalts bewirken können. Das jeweilige Material wird hierbei in fachüblichen Routinemassnahmen auf den jeweiligen Behälterinhalt abgestimmt. Typische Beispiele geeigneter Materialien sind Atemkalk (eine auch als Natronkalk bekannte Mischung aus Calciumhydroxid Ca(OH)₂ und Natriumhydroxid NaOH; oder auch eine Mischung aus Kaliumhydroxid KOH und Bariumhydroxid Ba(OH)₂); Partikelfilter; Molekularsiebe, Silicagel.

Das Filterelement kann lösbar ausgestaltet sein. Bevorzugt ist das Filterelement in einer Gebrauchsposition lösbar ausgestaltet. Somit kann das Filterelement ausgetauscht werden und der jeweiligen Situation angepasst werden.

Die Kanäle zur Zuführung- und Abführung von Luft in dem Verschluss können, zumindest an einer Anschlussseite zum Filterelement, besonders bevorzugt als weibliche Lueranschlüsse (mit einem Innenkonus) oder mit einem Gewinde ausgestattet sein. So kann im Falle eines abnehmbaren Filterelements auch ohne angeschlossenes Filterelement bspw. ein Schlauch für die Zufuhr von Schutzgas besonders einfach an dem Verschluss angebracht werden.

Das Filterelement kann weiterhin abnehmbar und bevorzugt austauschbar ausgebildet sein.

Somit ist eine Wahl des Filtermediums möglich und der Verschluss ist sehr vielseitig verwendbar.

Weiterhin kann das Filterelement einen Filterweg umfassen, der eine chemische Reaktion des Filtermaterials mit der das Filtermaterial durchströmenden Luft ermöglicht. Ein Filterweg ist hierbei im Rahmen der Anmeldung definiert als Abstand von Lufteinlass in den Filterelement sowie Luftauslass aus dem Filterelement. Abhängig vom Filtermaterial ist der notwendige Filterweg unterschiedlich.

Der Verschluss kann einen Datenträger umfassen, bevorzugt einen RFID-Chip.

Ein Datenträger ermöglicht die automatische Erkennung des Verschlusses sowie der Daten, die auf dem Datenträger gespeichert sind, wie beispielsweise Daten des zugehörigen Behälters sowie des Inhalts des Behälters. Dies führt zu einer sicheren Arbeitsweise, da wichtige Daten übertragen werden können. So kann beispielsweise der Inhalt des Behälters fehlerfrei erkannt werden. Des Weiteren können Daten wie beispielsweise Volumen, Haltbarkeitsdaten oder entnommene Mengen über den Datenträger festgestellt beziehungsweise auf diesem gespeichert werden. Dies führt zu qualitativ hochwertigen Resultaten. Der Datenträger kann optisch und/oder elektronisch ausgebildet sein.

Der Verschluss kann zumindest ein Fixierungselement umfassen, an welchem ein Adapter befestigbar, bevorzugt lösbar befestigbar, ist.

Ein derartiges Fixierungselement kann eine Hinterschneidung oder Kante oder Haltefläche umfassen, die von Eingriffselementen eines Adapters umgriffen werden können. Somit ist ein Adapter sicher auf dem Verschluss fixiert und positioniert.

Die Erfindung wird nachfolgend in Ausführungsbeispielen anhand von Figuren näher erläutert. Dabei zeigt:
- Fig. 1: Einen Schnitt durch einen erfindungsgemässen Verschluss in einer Lieferposition
- Fig. 2: Einen Schnitt durch einen erfindungsgemässen Verschluss in einer Gebrauchsposition
- Fig. 3: Einen Schnitt durch einen erfindungsgemässen Adapter in gekoppelter Position (Verschluss nicht gezeigt)
- Fig. 4: Einen Schnitt durch den Adapter aus Fig. 3 in entkoppelter Position (Verschluss nicht gezeigt)
- Fig. 5: Einen Schnitt durch eine erfindungsgemässe Versiegelungsvorrichtung
- Fig. 6: Einen Schnitt durch die Versiegelungsvorrichtung aus Fig. 5 mit geöffneten Siegelklappen
- Fig. 7: Einen Schnitt durch eine Versiegelungsvorrichtung gemäss Fig. 5 mit einem Durchstosselement in einer Lieferposition
- Fig. 8: Einen Schnitt durch eine Versiegelungsvorrichtung gemäss Fig. 7 in einer Gebrauchsposition
- Fig. 9: Eine Ansicht eines Anzeigeelements
- Fig. 10: Eine Schnittzeichnung durch einen Verschluss mit einem Behälter in Gebrauchsposition und einem entkoppelten Adapter
- Fig. 11: Eine Schnittzeichnung durch einen Verschluss mit einem Behälter in Gebrauchsposition und gekoppeltem Adapter.
- Fig. 12: Eine Schnittzeichnung durch die Mitte eines Verschlusses gemäss Figur 2 mit Kennzeichnung der Luftzufuhr
- Figur 13: Eine Schnittzeichnung durch einen Verschluss gemäss Fig. 12 durch eine zweite Schnittfläche (siehe Figur 14)
- Figur 14: Eine horizontale Schnittzeichnung eines Verschlusses gemäss Fig. 2 mit Darstellung der Schnittebene von Fig. 13.

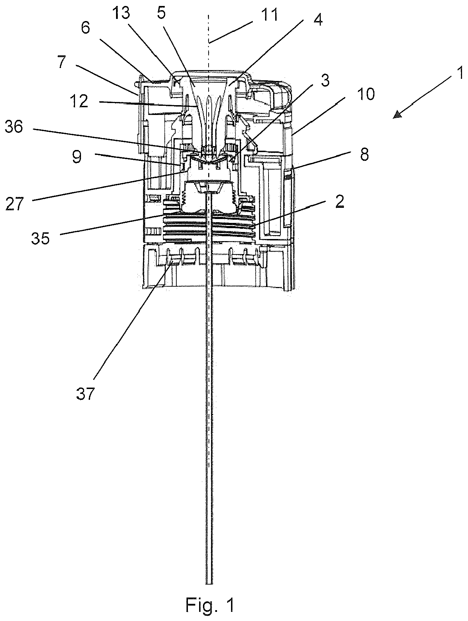

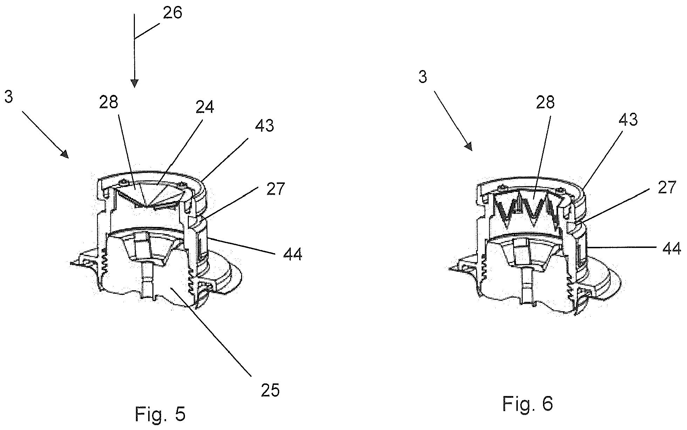

Figur 1 zeigt einen Schnitt durch einen erfindungsgemässen Verschluss 1 in einer Lieferposition. Der Verschluss 1 umfasst ein Verbindungselement 2 zu einem Behälter. Das Verbindungselement 2 wird durch ein Gewinde gebildet, welches in ein passendes Gegengewinde eines Behälters eingreifbar ist. Des Weiteren umfasst der Verschluss 1 eine Versiegelungsvorrichtung 3, die den Zugang zum Inneren des Behälters vor einer erstmaligen Nutzung versiegelt. Der Verschluss 1 umfasst weiterhin eine Schnittstelle 4, die mit einem Adapter verbindbar ist. Die Schnittstelle 4 umfasst eine Kupplungsaufnahme 5, in die ein Kupplungselement eines Adapters eingreifbar ist. Die Kupplungsaufnahme 5 stellt gleichzeitig ein Durchbruchselement zum Durchbrechen der Versiegelung der Versiegelungsvorrichtung 3 dar. Die Kupplungsaufnahme 5 ist entlang einer Längsachse 11 in Richtung Behälter verschiebbar ausgebildet und dringt so in die Versiegelungsvorrichtung 3 ein und bricht das Siegel auf. Gleichzeitig kann die Versiegelungsvorrichtung 3 eine weitere Sollbruchstelle umfassen (siehe Figur 5 und 6). Die Kupplungsaufnahme 5 umfasst weiterhin acht Eingriffsnuten 12, in die ein passendes Kupplungselement mit acht oder weniger Eingriffelementen eingreifen kann. Eine derartige Verbindung ermöglicht eine pass- und orientierungsgenaue und im Spiel reduzierte Verbindung zwischen einem Adapter und einem Verschluss 1. Die Schnittstelle 4 umfasst weiterhin Fixierungselemente 13 für einen Adapter. Unterhalb der Versiegelungsvorrichtung 3, in einem Kontaktbereich zwischen Behälteroberkante und Versiegelungsvorrichtung 3 ist ein Dichtungselement 35 angeordnet, welches durch einen PTFE-Folienring gebildet wird. Die Schnittstelle 4 umfasst weiterhin Perforationshilfen 36, die auf Sollbruchstellen der Versieglungsvorrichtung 3 ausgerichtet sind. Der Verschluss 1 umfasst ausserdem ein Deckelelement 6, welches klappbar auf dem Verschluss befestigt ist. Der Verschluss 1 umfasst weiterhin ein Bruchelement 7, welches ein erstmaliges Öffnen des Deckels anzeigt. Des Weiteren ist das Deckelelement 6 entfernbar ausgebildet. Durch ein derartiges Deckelelement 6, kann der Verschluss 1 auch nach dem Öffnen der Versiegelungsvorrichtung 3 wieder verschlossen werden. Der Verschluss 1 umfasst weiterhin ein Fixierungselement 10 für das Deckelelement, welches die Schnittstelle 4 vor der Benutzung fixiert. Nach dem Entfernen des Fixierungselements 10, welches durch Abreissen von dem Verschluss entfernt wird, kann die Kupplungsaufnahme 5 entlang der Längsachse 11 bewegt werden und somit die Versiegelungsvorrichtung 3 aufgebrochen werden. Neben der Versiegelung der Versiegelungsvorrichtung 3, die durch die Perforationshilfen 36 der Kupplungsaufnahme 5 aufgebrochen werden, umfasst die Versiegelungsvorrichtung 3, eine weitere Sollbruchstelle 27, die am Umfang der Versiegelungsvorrichtung 3 angeordnet ist. Durch das Aufbrechen dieser Sollbruchstelle 27, wird ein erster Teil der Versiegelungsvorrichtung 3 in Richtung Behälter entlang der Längsachse 11 verschoben. Durch diese Verschiebung werden Kanäle 9 geöffnet, durch die Luft in den Behälter zugeführt werden kann. Die Luft, die durch Kanäle 9 in den Behälter geführt werden kann, ist vorgängig durch Filterelement 8 gereinigt. Es besteht somit kein direkter Kontakt zwischen dem Inhalt des Behälters und der Aussenluft.

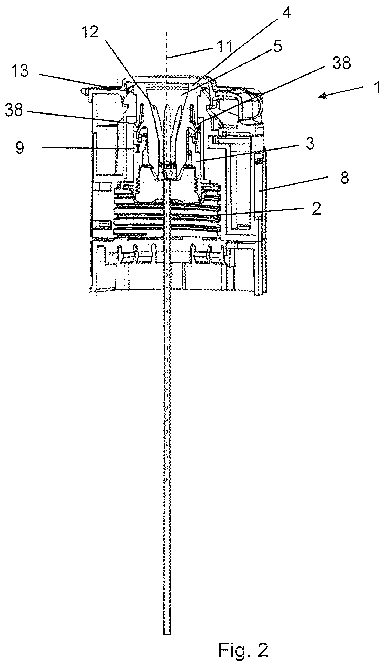

Figur 2 zeigt den Verschluss 1 aus Figur 1 in einer Gebrauchsposition. In der Gebrauchsposition ist die Kupplungsaufnahme 5 abgesenkt. Die Versiegelung der Versiegelungsvorrichtung 3 ist aufgebrochen und ein Zugang zum Inneren eines Behälters ist ermöglicht, so dass Fluid durch den Verschluss leitbar ist. Der Kanal 9 ist ebenfalls zugänglich, so dass Luft, welches durch das Filterelement 8 geführt worden ist, in den Behälter eindringen kann. Der Verschluss 1 weist weiterhin Schnapper 37 auf, die den Verschluss nach dem Aufschrauben auf einen Behälter fixieren. Somit kann der Verschluss nur schwer oder gar nicht von dem Behälter entfernt werden. Der Verschluss 1 weist weiterhin eine Schnittstelle 4 auf, mit einer Kupplungsaufnahme 5, in die Kupplungselement eines Adapters einführbar sind. Das Fixierungselement 10 (siehe Figur 1) ist in der Gebrauchsposition entfernt, und der Verschluss 1 weist somit eine geringere Höhenausdehnung als in der Lieferposition auf. Die Kupplungsaufnahme 5 weist einen Innen zumindest teilweise konisch zusammenlaufenden Bereich auf, so dass ein Kupplungselement eines Adapters leicht einführbar und zentrierbar ist. Des Weiteren sind an der Kupplungsaufnahme fünf Schnappelemente 38 ausgebildet, die die Kupplungsaufnahme 5 in der Gebrauchsposition fixieren; eine andere Anzahl von Schnappelementen 38 ist selbstverständlich möglich.

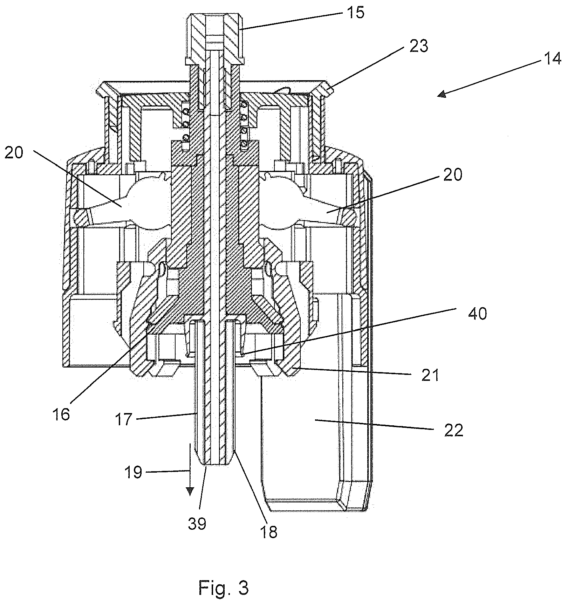

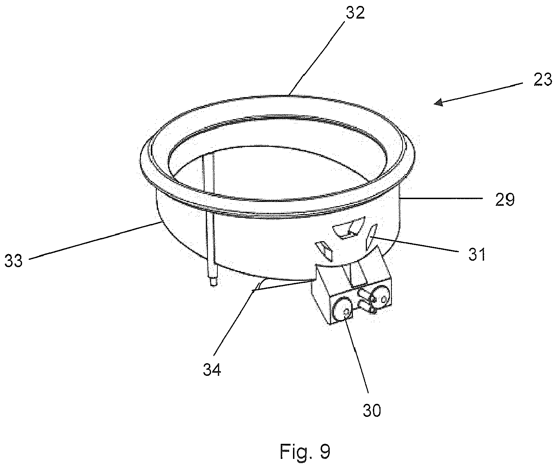

Figur 3 zeigt einen Schnitt durch eine Adapter 14 in gekoppeltem Zustand. Der Adapter 14 umfasst eine Schnittstelle 16 zu einem Verschluss sowie eine Verbindungsstelle 15 zu einem Gerät wie beispielsweise einer Auswertungseinheit. Die Schnittstelle 16 zu einem Verschluss (siehe Figur 1 und 2) umfasst ein Kupplungselement 17, welches Eingriffselemente 18 entlang seines Umfangs aufweist. Die Eingriffselemente 18 sind in Einführungsnuten 12 (siehe Figur 1) eines Verschlusses einführbar. Die Schnittstelle 16 ist in Einführungsrichtung 19 innerhalb des Adapters 14 verschiebbar ausgebildet. Das Kupplungselement 17 weist an seiner Spitze in Einführungsrichtung 19 eine Dichtfläche 39 auf, die eine Entnahme eines Fluids aus einem Behälter über einen Verschluss 1 ohne Leckagen, Verluste oder äussere Kontaminationen erlaubt. Des Weiteren sind Dichtungselemente 40 an dem Kupplungselement 17 ausgebildet, die einen hermetischen Luftabschluss bei einer Verbindung des Adapters 14 mit einem Verschluss ermöglichen. Die Bewegung des Kupplungselements 17 relativ zum Adapter 14 wird durch Hebelelemente 20 in seiner Ausdehnung begrenzt sowie gesteuert. Die Schnittstelle 16 zu einem Verschluss weist weiterhin Befestigungselemente 21 auf, die den Adapter 14 lösbar mit einem Verschluss 1 (siehe Figur 1 oder 2) verbinden. Der Adapter 14 weist weiterhin ein Ausleseelement 22 für Daten eines Verschlusses 1 (siehe Figur 1 oder 2) auf. Das Ausleseelement kann bevorzugt RFID-Daten auslesen. Somit können Daten eines Verschlusses direkt über den Adapter 14 an ein Gerät weitergegeben werden. Der Adapter 14 weist weiterhin ein Anzeigelement 23 (siehe auch Figur 9), welches die Verbindung von dem Adapter 14 mit einem Verschluss anzeigt. Das Anzeigelement 23 wird daher bei erfolgreicher Verbindung illuminiert. In Kenntnis der Erfindung ist ersichtlich, dass auch weitere Zustände angezeigt werden können (bspw. Fehlermeldungen; kein RFID gefunden; falsches RFID gefunden; Übersprechen mehrerer RFIDs; ablaufende shelf life in gekoppeltem Zustand; etc.)

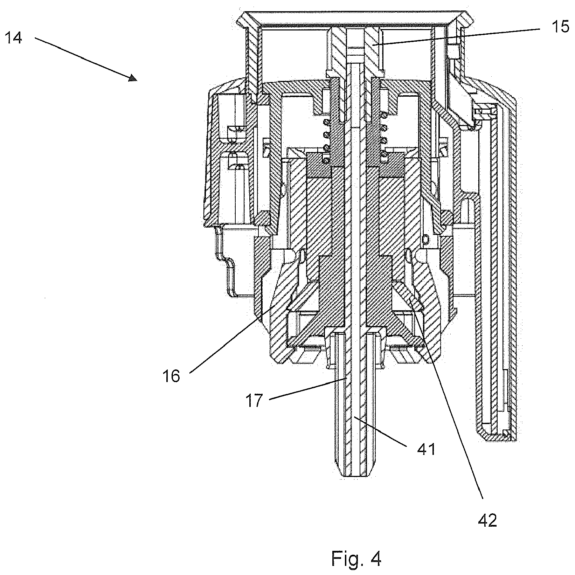

Figur 4 zeigt einen Schnitt durch einen Adapter 14 wie in Figur 3, jedoch in entkoppeltem Zustand. Der Adapter 14 ist analog zur Beschreibung der Figur 3 ausgebildet. In der entkoppelten ist jedoch die Schnittstelle 16 zu einem Verschluss in Richtung der Einführungsrichtung 19 abgesenkt. Die Schnittstelle 16 umfasst ein Kupplungselement 17, welches im Inneren einen rohrförmigen Hohlraum 41 zur Durchführung von Fluid aufweist. Dieser Hohlraum 41 erstreckt sich bis in die Verbindungsstelle 15, so dass das Fluid weiter in ein Gerät gefördert werden kann. Die Schnittstelle 16 weist weiterhin ein Rastelement 42 auf. Durch Betätigung des Rastelements 42 entgegen der Einführungsrichtung 19 werden die Befestigungselemente gespreizt und der Adapter 14 kann von dem Verschluss gelöst werden. Während dem Lösen des Adapters 14 vom Verschluss wird das Rastelement in Einführungsrichtung 19 gezwungen. Somit befindet sich der Adapter mit dem Rastelement nach dem Lösen wieder im entkoppelten Zustand.

Figur 5 zeigt einen Schnitt durch eine erfindungsgemässe Versiegelungsvorrichtung in einem versiegelten Zustand. Die Versiegelungsvorrichtung 3 weist ein Bruchelement 24 sowie ein Fluidanschlusselement 25 auf. Das Bruchelement 24 weist Siegelklappen 28 auf, die mittels eines Durchstosselements in eine Durchstossrichtung 26 durchbrochen werden können. Weiterhin ist die Versiegelungsvorrichtung 3 mit einer am Umfang angeordneten Sollbruchstelle 27 versehen. Die Sollbruchstelle 27 zerteilt das Bruchelement 24 in einen ersten Teil 43 und einen zweiten Teil 44. Eine solche Sollbruchstelle kann vorgesehen sein, ist jedoch nicht zwingend nötig.

Figur 6 zeigt die Versiegelungsvorrichtung 3 aus Figur 5 mit aufgebrochenen Siegelklappen 28. Die Siegelklappen 28 sind dreieckig ausgebildet, und bleiben entlang des Umfangs der Öffnung an der Versiegelungsvorrichtung 3 fixiert. Die Sollbruchstelle 27 ist in diesem Stadium noch nicht aufgebrochen. Zum Erreichen der vollständigen Gebrauchsposition wird auch die Sollbruchstelle 27 entlang des Umfangs der Versiegelungsvorrichtung 3 aufgebrochen und der erste Teil 43 der Versiegelungsvorrichtung 3 wird teilweise in den zweiten Teil 44 der Versiegelungsvorrichtung 3 eingeschoben.

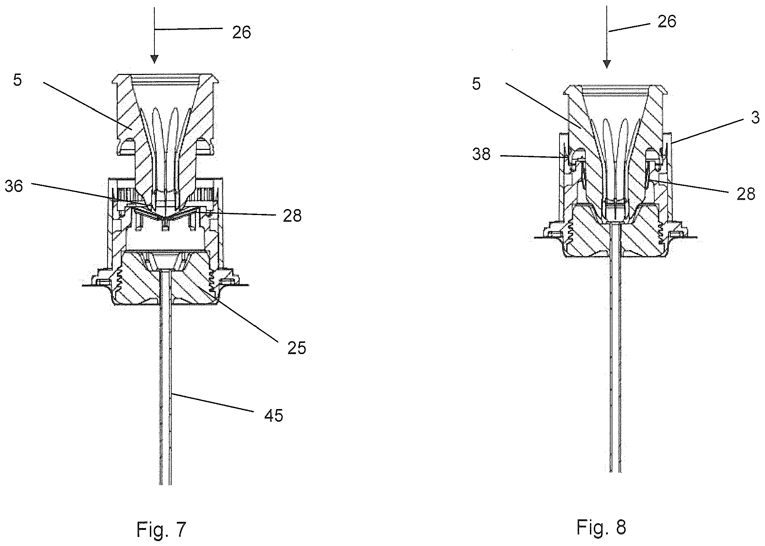

Figur 7 zeigt ein Schnitt durch eine Versiegelungsvorrichtung 3 gemäss Figur 5 mit einem Durchstosselement in eine Lieferposition. Das Durchstosselement wird durch die Kupplungsaufnahme 5 (siehe Figur 1 oder 2) gebildet und durchstösst die Siegelklappen 28 in Durchstossrichtung 26. Hierzu weist die Kupplungsaufnahme 5 an ihrer vorderen Spitze Perforationshilfen 36 auf, die passgenau jeweils zwischen zwei Siegelklappen aufsetzen und so die Siegelklappen aufbrechen. Zusätzlich zur in Figur 5 dargestellten Ausführungsform weist das Fluidanschlusselement 25 einen Entnahmeschlauch 45 auf, der bevorzugt bis zum Boden des Behälters reicht, in welchem der Verschluss mit der Versiegelungsvorrichtung angeordnet ist.

Figur 8 zeigt die Versieglungsvorrichtung 3 mit der Kupplungsvorrichtung 5 aus Figur 7 in einer Gebrauchsposition. Die Kupplungsaufnahme 5 ist in Richtung der Durchstossrichtung 26 verschoben und hat die Siegelklappen 28 aufgebrochen. Die Siegelklappen 28 bleiben an der Versiegelungsvorrichtung 3 befestigt liegen jedoch seitlich an der Kupplungsaufnahme an. Die Kupplungsaufnahme 5 weist weiterhin Schnappelemente 38 auf, die in Hinterschneidungen der Versiegelungsvorrichtung 3 eingreifen, so dass die Kupplungsaufnahme 5 in ihrer Position fixiert bleibt.

Figur 9 zeigt ein Anzeigelement 23, welches den Kupplungszustand von einem Adapter 14 (siehe Figur 3 und 4) und einem Verschluss 1 (siehe Figur 1 und 2) anzeigt. Das Anzeigelement 23 weist einen Anzeigekörper 29 und eine Lichtankopplungsvorrichtung 30 auf. Das eingekoppelte Licht, beispielsweise LED-Licht, wird durch die Lichtankopplungsvorrichtung 30 auf Lichtlenkelemente 31 gerichtet und durch die Lichtlenkelemente 31 im Anzeigekörper 29 verteilt. Die Lichtlenkelemente 31 sind Aussparungen in dem Anzeigekörper 29 der aus transparentem Polyethylenterephtalat (PET) besteht. Somit werden durch die Lichtlenkelemente 31 Grenzflächen gebildet und das eingekoppelte Licht wird reflektiert. Die Formen der Lichtlenkelemente 31 sind so ausgestaltet, dass das Licht möglichst gleichmässig im Anzeigekörper 29 verteilt wird. Das Licht wird somit im Wesentlichen um 90° umgelenkt, wobei die Umlenkflächen keine Graden darstellen sondern leicht gebogen ausgebildet sind. Somit wird eine breitere Streuung des Lichts innerhalb des Anzeigekörpers 29 erreicht. Der Anzeigekörper 29 weist eine Oberseite 32 und eine Unterseite 33 auf. Die Unterseite 33 ist im Vergleich zu Oberseite 32 um einen Winkel 34 geneigt ausgebildet. Der Winkel 34 beträgt 12°. Der Anzeigekörper 29 weist eine runde Form auf sowie ein flanschartige Ausstülpung an der Oberseite 32, um die Sichtbarkeit zu optimieren.

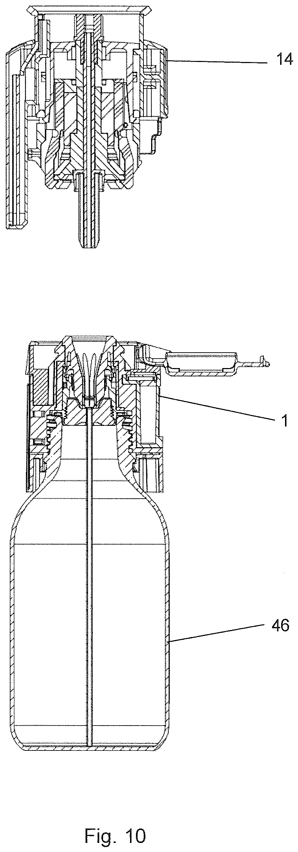

Figur 10 zeigt einen Schnitt durch eine einen Verschluss mit einem Behälter 46 in einer Gebrauchsposition und einem Adapter in entkoppeltem Zustand. Das Adaptersystem besteht aus einem Verschluss 1 (siehe Figur 1 und 2) und einem Adapter 14 (siehe Figur 3 und 4).

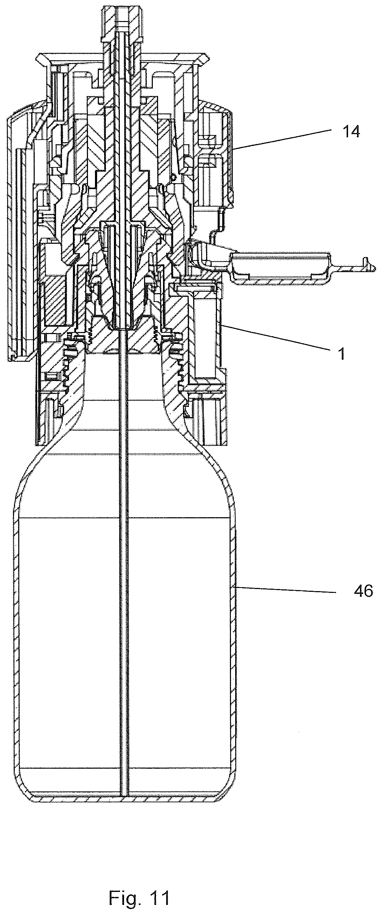

Figur 11 zeigt einen Schnitt durch ein Adaptersystem auf einem Behälter 46 in einer Gebrauchsposition.

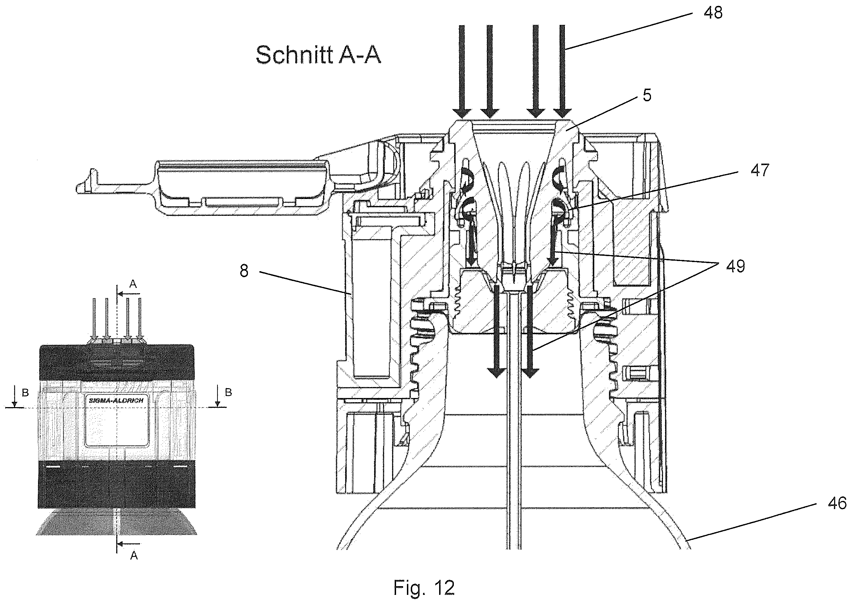

Figur 12, 13, 14 zeigen den Verschluss aus Figur 2 in Schnittdarstellungen mit Darstellung der Luftzufuhr durch den Verschluss 1. Aussenluft, wird jeweils so umgeleitet, dass keine Aussenluft direkt durch den Verschluss gelangen kann. Figur 12 zeigt die Aussenluft 48, die nur bis zu einem Dichtungselement 47 in den Verschluss eindringen kann. Von dort wird die Aussenluft 48 durch das Filterelement 8, welches insbesondere ein Absorberelement darstellt geleitet, so dass nur gereinigte Luft 49 in den Behälter 46 eindringen kann.

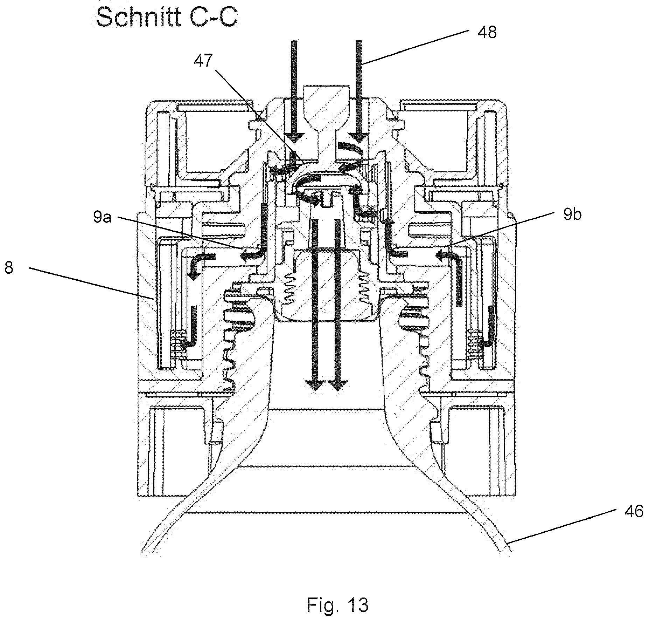

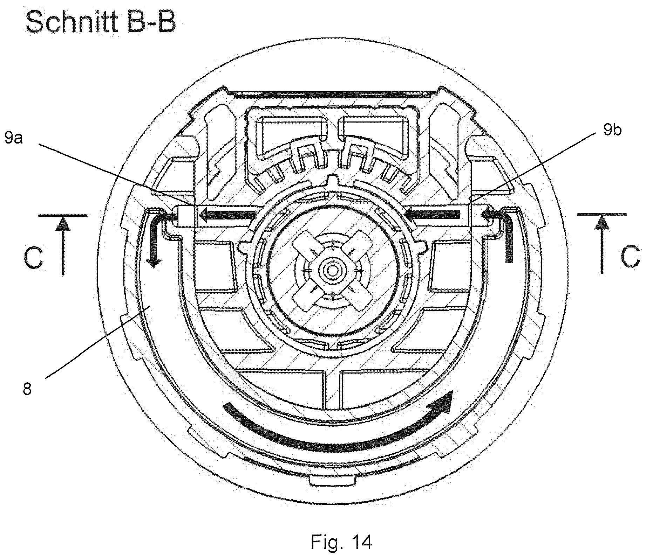

Figur 13 zeigt eine zweite Schnittfläche (bezüglich der Lage siehe Figur 14), durch die der Fluss der Luft genauer darstellbar ist. Die Aussenluft 48 wird durch Dichtungselemente 47 in einen seitlichen Kanal 9 a geleitet und von dort in das Filterelement 8, welches zumindest teilweise am Umfang des Verschlusses 1 angeordnet ist. Die Luft wird durch das Filterelement gefiltert und in Kanal 9 b eingeleitet, durch welchen die Luft in das Innere des Verschlusses 1 in den Behälter 46 geleitet wird.

Figur 14 zeigt einen horizontalen Schnitt durch den Verschluss 1 aus Figur 13. Der Kreislauf der Luft verläuft durch Kanal 9a in das Filterelement 8 am Umfang des Verschlusses 1 durch Kanal 9b in den Behälter 46 (siehe Figur 13).

Die folgenden Ausführungsformen sind möglich:
I. Verschluss (1) für einen Behälter insbesondere für Fluide, insbesondere Flüssigkeiten, umfassend ein Verbindungselement (2) zur Verbindung des Verschlusses mit einem Behälter, eine Versiegelungsvorrichtung (3) zum Versiegeln des Zugangs zu einem Behälterinhalt und eine Schnittstelle (4) zu einem Adapter mit einer Kupplungsaufnahme (5), wobei der Verschluss (1) eine Lieferposition und eine Gebrauchsposition aufweist, wobei in der Lieferposition die Versiegelungsvorrichtung (3) im Wesentlichen gas- und flüssigkeitsdicht verschlossen ist, und wobei in der Gebrauchsposition die Versiegelungsvorrichtung (3) geöffnet ist, dadurch gekennzeichnet, dass Kanäle (9) zur Zuführung und Abführung von Luft durch die Versiegelungsvorrichtung verschliessbar ausgebildet sind.
II. Verschluss (1) nach Ausführungsbeispiel I, dadurch gekennzeichnet, dass an dem Verschluss (1) ein Deckelelement (6) zum Öffnen und Verschliessen des Verschlusses (1) ausgebildet ist, wobei das Deckelelement (6) bevorzugt durch ein Bruchelement (7) sicherbar ist, welches ein erstmaliges Öffnen anzeigt.
III. Verschluss (1) nach einem der vorhergehenden Ausführungsbeispiele, dadurch gekennzeichnet, dass der Verschluss (1) ein Filterelement (8) umfasst, durch welches, insbesondere in der Gebrauchsposition der Versiegelungsvorrichtung (3) Aussenluft von Aussen durch den Verschluss (1) in einen Behälter leitbar ist.
IV. Verschluss (1) nach einem der vorhergehenden Ausführungsbeispiele, dadurch gekennzeichnet, dass der Verschluss (1) ein Fixierungselement (10) umfasst, welches den Verschluss (1) in einer Lieferposition fixiert, wobei das Fixierungselement (10) lösbar ausgebildet ist, bevorzugt zerstörbar lösbar ausgebildet und nach Lösung des Fixierungselements (10) der Verschluss (1) in eine Gebrauchsposition bringbar ist.
V. Verschluss (1) nach einem der vorhergehenden Ausführungsbeispiele, dadurch gekennzeichnet, dass der Verschluss (1) einen Datenträger umfasst, bevorzugt einen Datenträger mit zerstörbarem Datenübertragungselement, insbesondere bevorzugt ein RFID-Chip.
VI. Verschluss (1) nach einem der vorhergehenden Ausführungsbeispiele, dadurch gekennzeichnet, dass die Kupplungsaufnahme (5) entlang einer Längsachse (11) des Verschlusses (1) von einer Lieferposition in eine Gebrauchsposition bewegbar ausgebildet ist.
VII. Verschluss (1) nach Ausführungsbeispiel VI, dadurch gekennzeichnet, dass die Versiegelungsvorrichtung (3) durch die Bewegung der Kupplungsaufnahme (5) brechbar ist, wobei die Kupplungsaufnahme (5) nach dem Aufbrechen der Versiegelungsvorrichtung (3) in der Gebrauchsposition fixierbar ist.
VIII. Verschluss (1) nach Ausführungsbeispiel VI oder VII, dadurch gekennzeichnet, dass in der Gebrauchsposition gereinigte Luft durch den Verschluss (1) leitbar ist, insbesondere durch ein Filterelement.
IX. Verschluss (1) nach einem der vorhergehenden Ausführungsbeispiele, dadurch gekennzeichnet, dass die Kupplungsaufnahme (5) Eingriffsnuten (12) umfasst, bevorzugt acht Eingriffsnuten (12), die ausschliesslich eine Führungsfunktion für ein Kupplungselement eines Adapters aufweisen, insbesondere keine Haltefunktion zum Fixieren eines Adapters in einem gekoppelten Zustand von Verschluss und Adapter.
X. Verschluss nach einem der vorhergehenden Ausführungsbeispiele, dadurch gekennzeichnet, dass die Versiegelungsvorrichtung mindestens eine Sollbruchstelle aufweist, die bevorzugt ein simultanes Aufbrechen der Versiegelung der Kanäle zur Luftzu- und abfuhr sowie der Fluidversiegelung ermöglicht.
XI. Verschluss nach Ausführungsbeispiel X, dadurch gekennzeichnet, dass der Verschluss mindestens zwei Dichtungen umfasst, bevorzugt eine Dichtung zum Abschluss der Luftkanäle gegen Aussenluft und eine zweite Dichtung zum Abschluss des zu entnehmenden Fluids an einem Entnahmeschlauch (45).
XII. Verschluss (1), bevorzugt nach einem der vorhergehenden Ausführungsbeispiele, dadurch gekennzeichnet, dass er zur mehrfachen Verwendung ausgebildet ist und hierfür insbesondere keine von der Gebrauchsposition verschiedene Lieferposition aufweist, und wobei der Verschluss insbesondere auch kein Fixierungselement (10) aufweist.
XIII. Behälter, der mit einem Verschluss (1) nach einem der vorhergehenden Ausführungsbeispiele verbunden, bevorzugt im Wesentlichen gas- und flüssigkeitsdicht verschlossen ist.
XIV. Adapter (14) für einen Verschluss (1), bevorzugt einen Verschluss (1) nach einem der vorhergehenden Ausführungsbeispiele I bis XI, umfassend eine Verbindungsstelle (15) zu einem Gerät, bevorzugt einer Auswertungseinheit, und eine Schnittstelle (16) zu einem Verschluss (1), wobei die Schnittstelle (16) ein Kupplungselement (17) umfasst, welches mit einer Kupplungsaufnahme (5) eines Verschlusses (1) in Eingriff bringbar ist, so dass ein Fluidstrom durch den Adapter (14) erzeugbar ist, ohne einen direkten Kontakt von Aussenluft und Fluid, dadurch gekennzeichnet ,dass das Kupplungselement (17) relativ zum Adapter (14) entlang der Achse in einer Einführungsrichtung (19) des Adapters (14) in einen Verschluss (1) bewegbar, ausgebildet ist.
XV. Adapter nach Ausführungsbeispiel XIV, dadurch gekennzeichnet, dass das Kupplungselement (17) Eingriffselemente (18) umfasst, die in Eingriffsnuten einer Kupplungsaufnahme eines Verschlusses einführbar sind,.
XVI. Adapter (14) nach Ausführungsbeispiel XIV oder XV dadurch gekennzeichnet, dass das Kupplungselement (17) Eingriffselemente (18), bevorzugt acht Eingriffselemente (18), umfasst, die in Eingriffsnuten (12) einer Kupplungsaufnahme (5) einführbar sind, wobei die Eingriffsnuten bevorzugt an einem sich konisch in Richtung eines Behälters verjüngendem Bereich der Kupplungsaufnahme angeordnet sind.
XVII. Adapter (14) nach einem der Ausführungsbeispiele XIV bis XVI, dadurch gekennzeichnet, dass der Adapter (14) ein Ausleseelement (22) für Daten umfasst, bevorzugt eine Ausleseelektronik für einen RFID-Chip, und wobei das Ausleseelement (22) insbesondere zusätzlich eine Schreibfunktion für Daten aufweist.
XVIII. Adapter (14) nach einem der Ausführungsbeispiele XIV bis XVII, dadurch gekennzeichnet, dass der Adapter (14) ein Anzeigeelement (23) aufweist.

## Patentansprüche

1. Verschluss (1) für einen Behälter insbesondere für Fluide, insbesondere Flüssigkeiten, umfassend ein Verbindungselement (2) zur Verbindung des Verschlusses mit einem Behälter, eine Versiegelungsvorrichtung (3) zum Versiegeln des Zugangs zu einem Behälterinhalt und eine Schnittstelle (4) zu einem Adapter, und weiter umfassend eine Kupplungsaufnahme (5), wobei der Verschluss zur mehrfachen Verwendung ausgebildet ist, **dadurch gekennzeichnet, dass** der Verschluss (1) ein abnehmbares, bevorzugt austauschbares Filterelement (8) umfasst, durch welches Aussenluft von aussen durch den Verschluss (1) in einen Behälter leitbar ist.

2. Verschluss (1) nach Anspruch 1, **dadurch gekennzeichnet, dass** an dem Verschluss (1) ein Deckelelement (6) zum Öffnen und Verschliessen des Verschlusses (1) ausgebildet ist, wobei das Deckelelement (6) bevorzugt durch ein Bruchelement (7) sicherbar ist, welches ein erstmaliges Öffnen anzeigt.

3. Verschluss (1) nach einem der vorhergehenden Ansprüche, **dadurch gekennzeichnet, dass** der Verschluss (1) einen Datenträger umfasst, bevorzugt einen Datenträger mit zerstörbarem Datenübertragungselement, insbesondere bevorzugt ein RFID-Chip.

4. Verschluss (1) nach einem der vorhergehenden Ansprüche, **dadurch gekennzeichnet, dass** gereinigte Luft durch den Verschluss (1) leitbar ist, insbesondere durch das Filterelement (8).

5. Verschluss nach einem der vorhergehenden Ansprüche, wobei das Filterelement (8) einen Filterweg umfasst, der eine chemische Reaktion des Filtermaterials mit der das Filtermaterial durchströmenden Luft ermöglicht.

6. Verschluss nach einem der vorhergehenden Ansprüche, wobei das Filterelement (8) mit einem Material ausgestattet ist, welches dazu geeignet ist, Anteile aus der Umgebungsluft zu Filtern, welche eine Veränderung des jeweiligen Behälterinhalts bewirken können.

7. Verschluss (1) nach einem der vorhergehenden Ansprüche, **dadurch gekennzeichnet, dass** die Kupplungsaufnahme (5) Eingriffsnuten (12) umfasst, bevorzugt acht Eingriffsnuten (12), die ausschliesslich eine Führungsfunktion für ein Kupplungselement eines Adapters aufweisen, insbesondere keine Haltefunktion zum Fixieren eines Adapters in einem gekoppelten Zustand von Verschluss und Adapter.

8. Verschluss (1) nach einem der vorhergehenden Ansprüche, **dadurch gekennzeichnet, dass** der Verschluss (1) Kanäle zur Zuführung- und Abführung von Luft durch das Filterelement umfasst, wobei die Kanäle bevorzugt zumindest an einer Anschlussseite zum Filterelement als weibliche Lueranschlüsse oder mit einem Gewinde ausgestattet sind.

9. Verschluss nach einem der vorhergehenden Ansprüche, **dadurch gekennzeichnet, dass** der Verschluss mindestens zwei Dichtungen umfasst, bevorzugt eine Dichtung zum Abschluss der Luftkanäle gegen Aussenluft und eine zweite Dichtung zum Abschluss des zu entnehmenden Fluids an einem Entnahmeschlauch (45).

10. Behälter, der mit einem Verschluss (1) nach einem der vorhergehenden Ansprüche verbunden, bevorzugt im Wesentlichen flüssigkeitsdicht verschlossen ist.

11. Adapter (14) für einen Verschluss (1), bevorzugt einen Verschluss (1) nach einem der vorhergehenden Ansprüche 1 bis 9, umfassend eine Verbindungsstelle (15) zu einem Gerät, bevorzugt einer Auswertungseinheit, und eine Schnittstelle (16) zu einem Verschluss (1), wobei die Schnittstelle (16) ein Kupplungselement (17) umfasst, welches mit einer Kupplungsaufnahme (5) eines Verschlusses (1) in Eingriff bringbar ist, so dass ein Fluidstrom durch den Adapter (14) erzeugbar ist, ohne einen direkten Kontakt von Aussenluft und Fluid, **dadurch gekennzeichnet, dass** das Kupplungselement (17) relativ zum Adapter (14) entlang der Achse in einer Einführungsrichtung (19) des Adapters (14) in einen Verschluss (1) bewegbar, ausgebildet ist.

12. Adapter nach Anspruch 11, **dadurch gekennzeichnet, dass** das Kupplungselement (17) Eingriffselemente (18) umfasst, die in Eingriffsnuten einer Kupplungsaufnahme eines Verschlusses einführbar sind.

13. Adapter (14) nach Anspruch 11 oder 12 **dadurch gekennzeichnet, dass** das Kupplungselement (17) Eingriffselemente (18), bevorzugt acht Eingriffselemente (18), umfasst, die in Eingriffsnuten (12) einer Kupplungsaufnahme (5) einführbar sind, wobei die Eingriffsnuten bevorzugt an einem sich konisch in Richtung eines Behälters verjüngendem Bereich der Kupplungsaufnahme angeordnet sind.

14. Adapter (14) nach einem der Ansprüche 11 bis 13, **dadurch gekennzeichnet, dass** der Adapter (14) ein Ausleseelement (22) für Daten umfasst, bevorzugt eine Ausleseelektronik für einen RFID-Chip, und wobei das Ausleseelement (22) insbesondere zusätzlich eine Schreibfunktion für Daten aufweist.

15. Adapter (14) nach einem der Ansprüche 11 bis 14, **dadurch gekennzeichnet, dass** der Adapter (14) ein Anzeigeelement (23) aufweist.
